# EUROPEAN PATENT APPLICATION

(11) **EP 1 746 420 A1**
(43) Date of publication of application: **24.01.2007**
(21) Application number: 05741314.8
(22) Date of filing: 13.05.2005
(51) Int. Cl.: G01N 33/543

(54) **METHOD OF RAPIDLY AND EASILY DETECTING TARGET SUBSTANCE AND ENZYMOIMMUNOLOGICAL KIT**

(30) Priority: 14.05.2004 JP 2004144511
(71) Applicant: TORAY INDUSTRIES, INC., Tokyo 103-8666 (JP)
(72) Inventor: SHIBAYAMA, Naoko, 5250027 (JP); MIWA, Keishi, 1580094 (JP)
(74) Representative: Denison, Christopher Marcus
(86) International application number: PCT/JP2005/009217
(87) International publication number: WO 2005/111616

(57) **Abstract**

A measurement kit capable of rapidly and conveniently detecting one type or a plurality of types of target substance(s) simultaneously in a sample and a measurement method therefor are provided. Furthermore, with the kit or the measurement method, disease with which a target substance is associated is diagnosed. The enzyme immunological kit comprises: a support upon the surface of which a substance that recognizes a target substance is immobilized; an enzyme-labeled antibody against the target substance; and a substrate whose reaction product is water-insoluble after the enzyme-labeled antibody is caused to undergo color development; with which the target substance in a sample is detected based on color development on the support surface. Alternatively, the measurement method comprises 2 reaction steps: the incubation step and the color development step, by which a target substance in a sample is detected or the concentration of the target substance in the sample is measured through visual determination of color developed on the support surface after the color development reaction.

## Description

### Technical Field

The present invention relates to a measurement kit for rapidly and conveniently detecting one type or a plurality of types of target substance(s) and to a method using such measurement kit. In particular, the use of the kit and the method in detection of bacterial toxins or cytokines that are present in blood and the like is suitable for rapid diagnosis of diseases with which such bacterial toxins or cytokines are associated.

### Background Art

In the cases of diseases caused by infection or non-infectious diseases such as severe pancreatitis, burns, and injuries, serious systemic inflammatory reactions are observed. It is known that cytokine concentrations in patients' blood rise to high levels (hypercytokinemia) in such cases (Igaku No Ayumi (Journal of clinical and experimental medicine), 2001/1/6, Vol. 196, No. 1, Ishiyaku Publishers, Inc.). Cytokines are thought to be immune-related proteins that are normally produced for biophylaxis by various cells including immunocytes due to stimulation by infection, injuries, or the like. Cytokines are then released outside the cells to exert their action. It has been revealed that *in vivo* overproduction of cytokines is involved in tissue injuries or pathological conditions of various inflammatory diseases. Even when no obvious infection is confirmed, the immune system is excessively activated by superantigens, the toxins that are produced by *Staphylococcus aureus,* group A β-hemolytic streptococcus, and the like. Such cases can result in hypercytokinemia. Unlike conventional antigens, superantigens are compounds that bind to major histocompatibility antigen complex class II (hereinafter, abbreviated as MHC class II) on antigen-presenting cells without being subjected to processing in such cells. Superantigens thus form complexes with the MHC class II, so as to stimulate T cells having the specific Vβ region and then to abnormally activate the immune system. It is thought that such superantigens cause fervescence or anthema, pressure decrease, vomiting upon food poisoning, induction of autoimmune diseases, and the like. Dozens of types of superantigens have been confirmed, including Staphylococcal Enterotoxins A, B, C, D, E, H, and I (hereinafter abbreviated as SEA, SEB, SEC, SED, SEE, SEH, and SEI, respectively) that are *Staphylococcus aureus* exotoxins, Toxic Shock Syndrome Toxin-1 (hereinafter, abbreviated as TSST-1), Streptococcal Pyrogenic Exotoxins A and C (hereinafter, abbreviated as SPEA and SPEC, respectively), which are group A β-hemolytic streptococcus exotoxins, viral proteins, and plant proteins. Superantigens may be further specified in the future. Examples of diseases in which superantigens are involved include toxic shock syndrome (hereinafter, abbreviated as TSS) caused by TSST-1 and Neonatal TSS-like exanthematous disease (hereinafter abbreviated as NTED). Furthermore, it has also been suggested that superantigens are involved in scarlatina, fulminant infectious disease due to Streptococcus group A (Streptcoccal Toxic Shock Syndrome; hereinafter, abbreviated as STSS), Kawasaki's disease, which is acute systemic angiitis, or the like.

In the case of hypercytokinemia, it has been reported that organ failure is caused by systemic inflammation, and progress in inflammatory reactions results in death. Because of rapid progress of pathological conditions, patients' prognosis depends a great deal upon realization of rapid measurement of blood cytokine concentrations and decreases in such concentrations by treatment. In the case of TSS, it is important to prevent the severity from increasing by early diagnosis and early initiation of treatment. In the case of STSS, because of its rapid progress of pathological conditions, the disease results in outcomes ranging from necrosis of extremities and dismemberment for treating such necrosis to death, unless diagnosed early and appropriately treated.

However, currently, to measure patients' blood cytokine concentrations, collected specimens must be submitted to hospital examination rooms or external testing companies. At most examination rooms, cytokine concentrations are measured by the enzyme-linked immunosorbent assay method (hereinafter, abbreviated as ELISA method) that requires advanced and complicated techniques and appliances. Hence, considerable time and cost are required to obtain results. Measurement of superantigens for diagnosis of TSS or STSS also requires complicated techniques and much time. Therefore, a rapid and convenient measurement method capable of measuring cytokine concentrations at bedside within an extremely short time has been desired. To cope with this problem, concerning rapid diagnosis of Group A β-hemolytic streptococcus infection, Patent document 1 discloses a method for measuring Strep A antigen. However, the detection subject of this method is limited to group A β-hemolytic streptococcus and the superantigen concentration in a specimen cannot be measured by this method.

An object of the measurement method disclosed in Patent document 1 is to detect a target substance by causing formation of a conjugated complex composed of a substance that recognizes a target substance, the target substance, and a labeled antibody on a support and then detecting signals generated from the labeled antibody. There are many types of such measurement method. For example, the above ELISA also involves causing formation of a conjugated complex composed of a substance that recognizes a target substance, the target substance, and an enzyme labeled antibody on a support, causing an enzyme reaction to take place in a liquid system, causing color development, measuring the absorbance of a liquid, and then detecting the target substance. Furthermore, various immunochromatography methods are also known. An immunochromatography method also involves causing formation of a conjugated complex composed of a substance that recognizes a target substance, the target substance, and a labeled antibody on a support and then detecting the target substance. In the case of the ELISA method, a target substance can be accurately quantified, since absorbance is measured using an absorption spectrometer. However, rapid and easy measurement as described above is difficult with the ELISA method. On the other hand, since the measurement method disclosed in Patent document 1 and the immunochromatography method were originally developed for rapid and easy measurement, rapid and easy measurement of a target substance is possible with these methods. However, these methods focus on ease of use and are intended for qualitative analysis to visually determine if subjects are positive or negative. Hence, a target substance cannot be accurately quantified with these methods.

As described above, a measurement method that satisfies both the needs of rapid and easy measurement and of accurate quantification has never existed to date.
Patent document 1 JP Patent Publication (Kokai) No. 6-50973 A (1994)

### Disclosure of the Invention

The objects of the present invention are to address such problems in the above conventional technology and to provide a measurement kit capable of rapidly and conveniently detecting one type or a plurality of types of target substance(s) simultaneously, a measurement method therefor, and a method for diagnosing diseases with which target substances are associated by the use of the kit or the measurement method.

The present inventors have intensively studied a measurement method that can satisfy both the needs of rapid and easy measurement and of accurate quantification. Thus, the present inventors have discovered: that rapid and easy measurement can be performed by eliminating the need for complicated addition of reagents and realizing a smaller number of steps for measurement completion; that accurate visual quantification of signals requires establishment of a linear correlation between a target substance and the signal size that is generated from a conjugated complex composed of a substance that recognizes a target substance, the target substance, and a labeled antibody formed on a support; and that establishment of such linear correlation can be achieved by regulation of the amount of a substance (that recognizes a target substance) to be immobilized on a support and the amount of the labeled antibody within appropriate ranges.

As a result of intensive studies, the present inventors have discovered that when the concentration of a substance that recognizes a target substance in a solution ranges from 0.1 to 20µg/ml at the time when the solution containing such substance that recognizes the target substance is caused to come into contact with a support, the target substance can be detected with good sensitivity. The present inventors have also discovered that a further preferable concentration of a substance that recognizes a target substance in a solution ranges from 0.5 to 10µg/ml, in order to further increase detection sensitivity. Moreover, as a result of intensive studies, the present inventors have also discovered that when the concentration of an enzyme-labeled antibody against a target substance in a solution to be used in an incubation step ranges from 0.1 to 20µg/ml, the shortest incubation time can be realized without color development due to non-specific binding. Therefore, the present inventors have completed the present invention

The present invention has the following constitution to achieve the above objects.
(1) An enzyme immunological kit, which comprises: a support upon the surface of which a substance that recognizes at least 1 type of target substance is immobilized, following which the surface is treated to inhibit non-specific adsorption of non-target substances; an enzyme-labeled antibody against the at least 1 type of target substance; a substrate whose reaction product is water-insoluble after the at least 1 type of enzyme-labeled antibody is caused to undergo color development; and at least 1 container for receiving the aforementioned items; and an antibody-antigen reaction and a color development reaction are caused to take place in the container and the target substance in a sample is detected based on color development on the support surface on which the substance that recognizes the target substance is immobilized at a concentration of 2 cm² or more per ml of the sample.
(2) An enzyme immunological kit, which comprises: a support upon the surface of which a substance that recognizes at least 1 type of target substance is immobilized, following which such surface is treated to inhibit non-specific adsorption of non-target substances, and then undergoes a reaction of the at least 1 type of target substance with a known concentration on such surface; an enzyme-labeled antibody against the at least 1 type of target substance; a substrate whose reaction product is water-insoluble after the at least 1 type of enzyme-labeled antibody is caused to undergo color development; and at least 1 container for receiving the aforementioned items; and with which antibody-antigen and color development reactions are caused to take place in the container and the concentration of the target substance with an unknown concentration in a sample is measured through comparison of color development between a portion(portions) where the target substance with the known concentration reacts and portions other than such portion(portions) on the support surface on which the substance that recognizes the target substance is immobilized at a concentration of 2 cm² or more per ml of the sample.
(3) The enzyme immunological kit according to (2), by which, simultaneously with detection of the target substance in the sample based on color development on the support surface, the concentration of the target substance with the unknown concentration in the sample is measured through comparison of color development between the portion(portions) where the target substance with the known concentration reacts and the portions other than such portion(portions) on the support surface.
(4) The enzyme immunological kit according to any one of (1) to (3), wherein the substance that recognizes the target substance is immobilized on the surface of the support through contact of a solution containing the substance that recognizes the target substance with the support, and wherein the concentration of the substance that recognizes the target substance in the solution ranges from 0.1 to 20 µg/ml when immobilized.
(5) The enzyme immunological kit according to (4), wherein pH ranges from 6.5 to 8.0 when immobilized.
(6) The enzyme immunological kit according to (4) or (5), wherein the concentration of the enzyme-labeled antibody against the target substance ranges from 0. 1 to 20µg/ml.
(7) The enzyme immunological kit according to any one of (1) to (6), wherein the target-substance-recognizing substance immobilized on the support is immobilized in an amount that causes the establishment of a linear correlation between the concentration of the target substance in the sample and the strength of color development.
(8) The enzyme immunological kit according to any one of (1) to (7), wherein the substance that recognizes the target substance is an antibody against the target substance and the antibody is a polyclonal antibody.
(9) The enzyme immunological kit according to any one of (1) to (8), wherein the activity of the substance that recognizes the target substance is not lowered until 6 months after treatment with an antisepsis agent, pressure or heat treatment, or storage with an inert gas or in a vacuum state.
(10) The enzyme immunological kit according to any one of (1) to (9), wherein the support is rod-shaped, plate-shaped, or shaped so as to have a depressed area(depressed areas).
(11) The enzyme immunological kit according to any one of (1) to (10), wherein the enzyme-labeled antibody is a monoclonal antibody.
(12) The enzyme immunological kit according to (11), wherein the monoclonal antibody is an antibody against a human interleukin.
(13) The enzyme immunological kit according to (11) or (12), wherein the monoclonal antibody is IgG.
(14) The enzyme immunological kit according to (13), wherein the monoclonal antibody is IgGl.
(15) The enzyme immunological kit according to any one of (12) to (14), wherein the human interleukin is IL-6 or IL-8.
(16) The enzyme immunological kit according to (15), wherein the monoclonal antibody recognizes a fragment comprising the 149^{th} to the 173^{rd} amino acids of the amino acid sequence of human IL-6.
(17) The enzyme immunological kit according to (15), wherein the monoclonal antibody recognizes a fragment comprising the 153^{rd} to the 162^{nd} amino acids of the amino acid sequence of human IL-6.
(18) The enzyme immunological kit according to (16), wherein the antibody against human IL-6 is a clone IG61 produced by the hybridoma deposited under accession No. FERM P-10713 with the International Patent Organism Depositary (IPOD), the National Institute of Advanced Industrial Science and Technology (AIST).
(19) The enzyme immunological kit according to (17), wherein the antibody against human IL-8 is a clone EL139 produced by the hybridoma deposited under accession No. FERM P-12710 with the International Patent Organism Depositary (IPOD), the National Institute of Advanced Industrial Science and Technology (AIST).
(20) The enzyme immunological kit according to any one of (1) to (19), wherein the enzyme for the enzyme-labeled antibody is horseradish peroxidase, alkaline phosphatase, or β-D-galactosidase.
(21) The enzyme immunological kit according to any one of (1) to (20), wherein the enzyme for the enzyme-labeled antibody is horseradish peroxidase and the substrate is a solution containing 3-amino-9-ethylcarbazole, 5-aminosalicylic acid, 4-chloro-1-naphthol, o-phenylenediamine, 2,2'-azino-bis(3-ethylbenzothiazolin-6-sulfonic acid), 3,3-diaminobenzidine, 3,3', 5,5'-tetramethylbenzidine, o-dianisidine, or 3,3-dimethoxybenzidine.
(22) The enzyme immunological kit according to any one of (1) to (21), wherein the enzyme for the enzyme-labeled antibody is alkaline phosphatase and the substrate is a solution containing 5-bromo-4-chloro-3-indryl phosphate, nitroblue tetrazolium, or p-nitrophenyl phosphate.
(23) The enzyme immunological kit according to any one of (1) to (20), wherein the enzyme for the enzyme-labeled antibody is β-D-galactosidase and the substrate is a solution containing either o-nitrophenyl-β-D-galactoside or 5-bromo-4-chloro-3-indole-β-D-galactopyranoside.
(24) The enzyme immunological kit according to any one of (1) to (23), wherein the target substance is a cytokine, *Staphylococcus aureus* exotoxin, or Streptococcus group A exotoxin.
(25) The enzyme immunological kit according to (24), wherein the cytokine is an inflammatory cytokine.
(26) The enzyme immunological kit according to (25), wherein the inflammatory cytokine is IL-1, IL-2, IL-4, IL-6, IL-8, TNF-α, or IFN-γ.
(27) The enzyme immunological kit according to any one of (24) to (26), wherein the detection limit concentration of the cytokine is 500 pg/ml.
(28) The enzyme immunological kit according to (24), wherein the *Staphylococcus aureus* exotoxin is a superantigen.
(29) The enzyme immunological kit according to (28), wherein the superantigen is SEA, SEB, SEC, SED, SEE, SEH, SEI, SEJ, or TSST-1.
(30) The enzyme immunological kit according to (24), wherein the Streptococcus group A exotoxin is a superantigen.
(31) The enzyme immunological kit according to (30), wherein the superantigen is SPEA, SPEC, SPEF, SPEG, or SPEH.
(32) The enzyme immunological kit according to any one of (1) to (27), which is used for rapidly determining whether a disease is caused by hypercytokinemia.
(33) The enzyme immunological kit according to any one of (1) to (24), (28), and (29), which is used for rapidly determining whether a disease is caused by *Staphylococcus aureus.*
(34) The enzyme immunological kit according to any one of (1) to (24), (30), and (31), which is used for rapidly determining whether a disease is caused by group A β-hemolytic streptococcus.
(35) The enzyme immunological kit according to any one of (1) to (35), which comprises converting color development on the support surface into numerical values using a measurement apparatus and then quantifying the color development using the numerical values, wherein light reflecting from the support surface is dispersed within a range between 400 nm and 700 nm, the reflectance at every each wavelength is measured, and parameters based on the XYZ or L*a*b* color system are thus measured.
(36) A method for detecting a target substance in a sample or measuring the concentration of a target substance in a sample, wherein the enzyme immunological kit according to any one of (1) to (35) is used.
(37) A method for detecting a target substance in a sample, which comprises 2 reaction steps: the incubation step for simultaneously incubating a support having a portion(portions) coated with a substance that recognizes a target substance, a sample, and an enzyme-labeled antibody against the target substance; and the color development step for causing color development to take place using a substrate whose reaction product is water-insoluble, wherein in the incubation step, the substance that recognizes the target substance is added to the support surface at a concentration of 2 cm² or more per ml of the sample, and the color of the support surface is visually determined after the color development reaction.
(38) The method for detecting a target substance in a sample according to (37), wherein the concentration of the substance that recognizes the target substance in the solution ranges from 0.1 to 20µg/ml and the pH ranges from 6.5 to 8.0 when the substance is immobilized on the support having a portion(portions) coated with the substance that recognizes the target substance.
(39) The method for detecting a target substance in a sample according to (37) or (38), wherein the concentration of the enzyme-labeled antibody against the target substance, which is used in the color development step, ranges from 0.1 to 20 µg/ml.
(40) The method according to any one of (37) to (39), which is a method for measuring the concentration of a target substance in a sample, wherein the color of the support surface is visually determined after the color development reaction.
(41) The method according to any one of (37) to (39), which is a method for detecting a target substance in a sample, wherein the color of the support surface is converted into numerical values using an apparatus after the color development reaction, and then the color development is quantified using the numerical values.
(42) The method according to any one of claims 36 to 41, which is a method for detecting a target substance in a sample, wherein light reflecting from a sample is dispersed within a range between 400 nm and 700 nm, the reflectance is measured at every each wavelength, and then parameters based on a color system are measured, and the color system is the XYZ or L*a*b* system.
(43) The method for detecting a target substance in a sample according to any one of (37) to (42), which comprises a washing step between the incubation step and the color development step.
(44) The method for detecting a target substance in a sample according to any one of (37) to (43), wherein the reaction temperature for the incubation, washing, or color development step is within the range between 4°C and 40°C.
(45) The method for detecting a target substance in a sample according to (44), wherein the incubation step, the color development step, and the washing step are performed at room temperature.
(46) The method for detecting a target substance in a sample according to any one of (37) to (45), wherein the amount of a sample is 200µl or less.
(47) The method for detecting a target substance in a sample according to any one of (37) to (46), wherein the time spent for the incubation step is within the range between 5 and 60 minutes and the time spent for the color development step is within the range between 5 and 30 minutes.
(48) The method for detecting a target substance in a sample according to any one of (37) to (47), which comprises causing at least 1 type of target substance with a known concentration to react with at least either a part of a portion(portions) coated with or a part of aportion(portions) not coated with the substance that recognizes the target substance on the support having such portion(portions) coated with the substance that recognizes the target substance, and then comparing color development between a portion(portions) where the target substance with the known concentration reacts and portions other than such portion(portions), thereby measuring the concentration of the target substance with an unknown concentration in a sample.
(49) The method according to any one of (37) to (48), wherein the support having portions coated with the substances that recognizes target-substances is coated with the target-substance-recognizing substance at at least two different concentrations and the concentration of a target substance in a sample is measured by comparing the degree of color development between at least 2 portions.
(50) The method according to any one of (37) to (49), wherein the target substance is a cytokine and the detection limit concentration thereof is 500 pg/ml.
(51) A method for diagnosing hypercytokinemia through detection of a cytokine using the enzyme immunological kit according to any one of (24) to (27) and (32).
(52) A method for diagnosing a disease caused by *Staphylococcus aureus* through detection of *Staphylococcus aureus* exotoxin using the enzyme immunological kit according to any one of (28), (29), and (33).
(53) A method for diagnosing a disease caused by group A β-hemolytic streptococcus through detection of group A β-hemolytic streptococcus exotoxin using the enzyme immunological kit according to any one of (30), (31), and (34).

This description includes part or all of the contents as disclosed in the description and/or drawings of Japanese Patent Application No. 2004-144511, which is a priority document of the present application.

### Brief Description of the Drawings

Fig. 1 is a photograph showing color development on supports resulting from the use of human plasma samples supplemented with IL-8.
Fig. 2 is a photograph showing color development on supports resulting from the use of human plasma samples supplemented with IL-6.
Fig. 3 is a photograph showing color development (quantification based on the L*a*b*color system) of supports resulting from the use of IL-8 in a patient's serum.
Fig. 4 shows a step schematic diagram that is an embodiment of the measurement method of the present invention.

### Best Mode of Carrying Out the Invention

The present invention relates to a measurement kit capable of rapidly and conveniently detecting a target substance, a measurement method for rapidly and conveniently detecting a target substance, and a method for diagnosing a disease with which a target substance is associated.

The present inventors have discovered a method capable of rapidly and conveniently detecting the concentration of one type or a plurality of types of target substance(s) by the use of a method that involves incubating a support on which a substance that recognizes a target substance is immobilized, an enzyme-labeled antibody against the target substance, and a sample to be measured and then causing color development to take place on the support using a substrate whose reaction product is water-insoluble after the enzyme-labeled antibody is caused to undergo color development.

In the present invention, "substance that recognizes a target substance" means a substance capable of binding to a labeled substance. Examples of such substance include an antibody against a target substance, a receptor or a ligand when it is in a receptor-ligand relationship with a target substance, and an enzyme, substrate, or the like binding to a target substance. Preferably, an antibody having strong binding force is used.

Furthermore, "incubation" in the present invention means to allow the relevant subjects to stand at a constant temperature during an antigen-antibody reaction. An "antigen-antibody reaction" means a reaction whereby an antibody specifically binds to an antigen having a structure complementary thereto. In the present invention, such antigen-antibody reaction refers to the binding of a substance that recognizes a target substance to the target substance.

Furthermore, "washing" in the present invention means to wash and remove substances that have not performed any antigen-antibody reaction in the incubation step. Washing materials are not particularly limited, as long as they have washing effects and have no effects on substances that bind as a result of an antigen-antibody reaction. To enhance washing effects, a surfactant may be added. In addition, washing methods are not particularly limited, as long as they do not directly damage a support.

Reaction temperatures employed in each of the incubation, washing, and color development steps of the present invention are not particularly limited. Such reaction temperatures are preferably within a range between 4°C and 40°C, so as not to cause freezing or evaporation of a reaction solution or deactivation of an enzyme. Reaction temperatures are most preferably within the range between 15°C and 40°C in order to activate an enzyme and to rapidly complete the reaction.

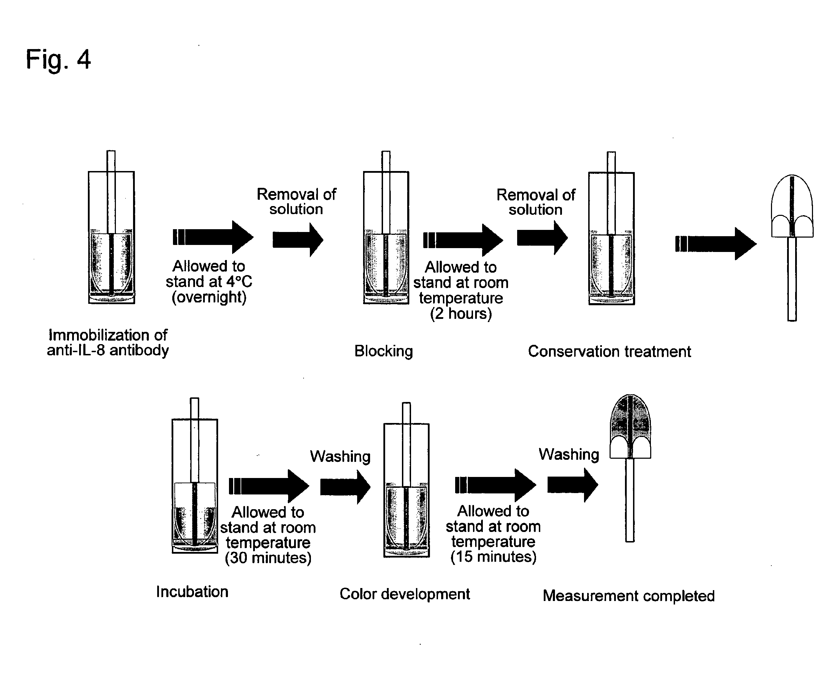

In the incubation step of the present invention, the longer the incubation time, the higher the reaction rates between a target substance and a substance that recognizes the target substance and between the target substance and an enzyme-labeled antibody against the target substance. As a result, the degree of the resulting color development is also increased. After a certain time, the antigen-antibody reaction reaches a saturation state. Moreover, the incubation time should be shortened so that measurement may be rapidly completed. The time spent for the incubation step can be shortened by: increasing the ratio of the amount of a sample to the area of a portion(portions) of the support on which a substance that recognizes a target substance has been immobilized; increasing the concentration of an enzyme-labeled antibody against a target substance in a solution that is used in the incubation step; or increasing the density at which a substance that recognizes a target substance is immobilized on a support. A substance that recognizes a target substance can be immobilized on the surface of a support by causing a solution containing the substance that recognizes the target substance to come into contact with the support. The higher the amount of an antibody binding to a support, the greater the amount of an antigen can be adsorbed. Hence, sensitivity can be improved and a shorter reaction time can be realized. However, when an antigen is present in an amount that blocks portions to which the antigen can be adsorbed, molecules mutually block reaction portions so as to lower adsorption efficiency. Hence, it is required to optimize the immobilization amount. As a result of intensive studies, the present inventors have discovered that a target substance can be detected with good sensitivity when the concentration of a substance that recognizes the target substance in a solution ranges from 0.1 to 20µg/ml when the solution containing the substance that recognizes the target substance contact with a support. The present inventors have discovered that to further increase detection sensitivity, it is further preferable to employ a concentration of a substance that recognizes a target substance in a solution ranging from 0.5 to 10µg/ml. A higher ratio of the amount of a sample to the area of a portion(portions) of a support, on which a substance that recognizes a target substance has been immobilized, results in more opportunities for the immobilized substance to come into contact with the target substance. Accordingly, a shorter incubation time can be realized. Specifically, on a support, the area of a portion(portions) of the support on which a substance that recognizes a target substance has been immobilized per ml of a sample is 2cm² or more per ml of the sample. That is, such substance that recognizes a target substance is immobilized on the above support surface at a concentration of 2cm² or more per ml of the sample. In measurement of the present invention, when an antibody-antigen reaction is caused to take place in a container, incubation is performed while immersing a portion(portions) of a support on which a target substance has been immobilized in a sample. At this time, the amount of the sample and/or the size of a container to be subjected to immobilization and addition is adjusted, so that 2cm² or more of the portion(portions) of the support on which the substance that recognizes the target substance has been immobilized, is immersed per one ml of the sample. For example, Fig. 4 is a step schematic diagram that is an embodiment of the measurement method of the present invention. In the example shown in Fig.4, incubation is performed by adding typically 250µl of a sample to a container. In this case, when a support on which a target substance has been immobilized is immersed, at least 0.5 cm² of a portion(portions) on which the target substance has been immobilized is immersed in a sample. An excessively high concentration of an enzyme-labeled antibody against a target substance in a solution to be used in the incubation step results in enhanced color development due to non-specific binding to a support or an immobilized substance. Hence, it is preferable to employ an optimum concentration that realizes the shortest incubation time and does not cause any color development due to non-specific binding. As a result of intensive studies, the present inventors have discovered that a concentration of an enzyme-labeled antibody against a target substance in a solution to be used in the incubation step ranging from 0.1 to 20µg/ml can realize the shortest incubation time without color development due to non-specific binding. In the present invention, the time spent for the incubation step is preferably between 5 and 60minutes and most preferably between 15 and 60minutes under conditions where: the area of a portion(portions) of a support on which a substance that recognizes a target substance has been immobilized is 2cm² or more per ml of the sample; the concentration of the substance that recognizes the target substance in a solution ranges from 0.1 to 20µg/ml when the solution containing the substance that recognizes the target substance is caused to come into contact with such support; and the concentration of an enzyme-labeled antibody against the target substance in the solution to be used in the incubation step ranges from 0.1 to 20µg/ml.

In the color development step of the present invention, the longer the time spent for the color development step, the higher the degree of color development. However, spending an excessively long time for color development causes the color development substrate itself to undergo color development due to its own natural decomposition. In the present invention, the time spent for the color development step is preferably between 5 and 30minutes, and most preferably between 15minutes and 30minutes under conditions where: the area of a portion(portions) of a support on which a substance that recognizes a target substance has been immobilized is 2cm² or more per ml of the sample; the concentration of the substance that recognizes the target substance in a solution ranges from 0.1 to 20µg/ml when the solution containing the substance that recognizes the target substance is caused to come into contact with the support; the concentration of an enzyme-labeled antibody against the target substance in a solution to be used in the incubation step ranges from 0.1 to 20µg/ml; and the time spent for the incubation step is between 5 and 60minutes. Moreover, to increase opportunities for an enzyme bound to a support through an antigen-antibody reaction to come into contact with a color development substrate and to realize a shorter time for color development, a color development substrate is applied to a portion(portions) to which the enzyme-labeled antibody has bound, preferably at a concentration of 2cm² per ml of the substrate.

And, "color development" in the present invention refers to the addition of a reagent for detection of a substance for a target color to be developed. With the measurement method and the enzyme immunological kit of the present invention, the degree of color development can be visually and directly confirmed. With some apparatuses, parameters based on a color system are measured so that color development can also be quantified using the same. Specifically, in the present invention, a target substance can be quantified by measuring reflection light.

"Color systems" mean methods for expressing the colors of subject substances and the colors of light sources with numerical values or symbols. Of these, an L*a*b*color system is one with uniform color spaces as specified by the Commission Internationale de l'Eclairage (CIE) and also employed for JIS (JIS Z8729). The L*a*b*color system involves calculation(s) based on X, Y, and Z, the tristimulus values, of an XYZ color system, regarding a sample. The XYZ color system is based on the principle of the additive mixture of color concerning the three primary colors of light, which is currently the base for each color system of the standard CIE color systems. According to the XYZ color system, colors are represented using a chromaticity diagram and the 3 values of Y, x, and y. "Y" corresponds to lightness based on reflectance and "x" and "y" correspond to chromaticity. According to the L*a*b*color system, lightness is represented using L*, and chromaticity representing hue and chromaticness is represented using a* and b*. With a larger L*, whiteness increases. With a smaller L*, blackness increases. "a*" represents the red direction, "-a*" represents the green direction, "b*" represents the yellow direction, and "-b*" represents the blue direction. The larger their absolute numerical values, the more vivid the colors; the lower their absolute numerical values, the more dull the colors. Hence, the L*a*b*color system is thought to represent color sensation closest to human color sensation. In the present invention, color system parameter measurement using an apparatus is not limited, as long as the degree of color development is converted into numerical values using the apparatus so that color development can be quantified using such values. Specifically, any type of measurement can be performed, as long as light reflecting from a sample is dispersed within the visible range between 400 nm and 700 nm, the reflectance at every each wavelength is measured, and then parameters based on a color system can be calculated. With the use of the results obtained by such reflectance measurements, parameters based on the XYZ color system, parameters based on the L*a*b* color system, and the like can be calculated. Reflectance measurement can be performed using a commercial colorimeter, for example.

At this time, when measurement is performed under the above conditions, the concentration of a target substance in a sample within a certain range has a linear correlation with values such as L*, a*, and b* calculated from spectral reflection factors or with a value such as ΔE*(ab) that can be calculated from ΔL*, Δa*, and Δb* (color differences obtained from coordinates L*, a*, and b* based on the L*a*b* color system). Therefore, the concentration of the target substance in the sample can be quantified accurately. Moreover, values that are converted into numerical values by the L*a*b* method represent degrees of lightness and chromaticness close to those determined by human color sensation. Therefore, the values are characterized by having high correlation with results obtained by rapid visual determination, and by having high accuracy. When rapid measurement and accurate quantification are performed simultaneously, the measurement method is superior to quantification methods using absorbance.

Hereinafter, the enzyme immunological kit of the present invention will be described in detail, and the measurement method of the present invention will also be explained.

The enzyme immunological kit of the present invention is used for detecting a target substance in sample. "Detect(ion)" used herein refers to the confirmation of the presence of a target substance. Quantification or semi-quantification of the concentration of a target substance is also included within such scope. A sample containing a target substance is preferably a solution that flows easily to realize smooth antigen-antibody reactions among a target substance, a substance immobilized on a support, and an enzyme-labeled antibody against the target substance. However, it is not particularly required that such sample be a liquid. For example, when a sample is a solid, it may be dissolved using an appropriate solvent or dispersed in a solvent. Furthermore, the amount of a sample required for measurement is not particularly limited, as long as it enables the sample to come into contact with a portion(portions) of a support on which a substance that recognizes a target substance has been immobilized. In many cases, the amounts of samples are small, because of limited collection amounts of blood, for example. In such cases, a sample is diluted, so that the amount of such sample can be increased. However, solely lowering the concentration of a target substance results in a decreased number of opportunities for a target substance, a substance immobilized on a support, and an enzyme-labeled antibody against the target substance to come into contact with each other per unit time. The thus slower reaction can result in difficulties in visual detection or quantification of the target substance or difficulties in detection or quantification of the target substance using a color system measurement apparatus. Incubation time can be prolonged to avoid a decreased total reaction amount. In view of realizing rapid measurement, the following method is preferably employed. Specifically, with a low sample amount, as already described above, the ratio of the amount of a sample to the area of a portion(portions) of a support on which a substance that recognizes a target substance has been immobilized is increased. Alternatively, the concentration of an enzyme-labeled antibody against a target substance in a solution in the incubation step is increased. Further alternatively, the immobilization density of a substance that recognizes a target substance on a support is increased. Furthermore, measurement is desirably performed using a sample in an amount that is as low as possible and enables easy handling, such as 10µl to 200µl.

In the present invention, coating with or immobilization of a substance that recognizes a target substance means to bind such substance that recognizes a target substance to a support via a covalent bond, metal bond, ionic bond, hydrophobic bond, hydrogen bond, hydrophilic bond, or the like. After the immobilization, treatment is performed to inhibit non-specific adsorption of the target substance, thereby preventing the target substance from being adsorbed to portions on which the substance that recognizes the target substance has not been immobilized. Therefore, the target substance can be efficiently detected. As a treatment method for inhibiting non-specific adsorption, a method referred to as blocking can be employed, by which gap portions where a substance that recognizes a target substance has not been bound are covered with another substance. When a substance that recognizes a target substance is a protein, blocking is preferably performed using a solution containing casein or bovine albumin that is expected to maintain the three-dimensional structure of immobilized molecules.

Furthermore, in the present invention, after a support is coated with substances that recognize a plurality of target substances with different concentrations, the target substances in a sample are caused to perform antigen-antibody reactions, so as to detect the target substances. The target substances in the sample can then be detected based on color development. Furthermore, through comparison of color development between a portion(portions) where a target substance with a known concentration reacts on the support surface and a portion(portions) other than such portion(portions), the concentration in the sample can also be measured. Moreover, the concentration of a target substance with an unknown concentration in a sample can also be measured by detecting the target substance in a sample based on color development on the support surface and simultaneously comparing color development between a portion(portions) where the target substance with a known concentration reacts on the support surface and a portion(portions) other than such portion(portions).

Examples of a "substance that recognizes a target substance" include an antibody against a target substance, a receptor or a ligand when it is in a receptor-ligand relationship with a target substance, and an enzyme or a substrate that binds to a target substance. Preferably, an antibody having strong binding ability is used.

A target substance in the present invention, that is, a substance to be detected, is not particularly limited. Examples of such target substance include biomolecules such as a protein, a peptide, and a lipid and non-biological substances such as a drug, a pigment, and a heavy metal. When a method that involves capturing a target substance using 2 types of antibodies is employed, a target substance preferably has a molecular weight corresponding to or higher than that of a peptide comprising 12 to 15amino acids. A cytokine as a target substance in the present invention is not particularly limited. Examples of such cytokine include, but are not limited to, cytokines derived from various animals such as a human, a mouse, a rabbit, and a pig and cytokines produced by cultured cells. *Staphylococcus aureu*s exotoxin and group A β-hemolytic streptococcus exotoxin as target substances in the present invention are not limited, as long as they are substances produced by the relevant bacteria. Preferably, such exotoxins are substances that are generally classified as superantigens, which overactivate T cells regardless of immunological specificity through their binding to the outside of T cell receptor (hereinafter, abbreviated as TCR) Vβ together with MHC class II molecules without being processed by antigen-presenting cells.

The material used for a support of the present invention is not particularly limited. However, for a portion(portions) on which a substance that recognizes a target substance is immobilized, a material having affinity for the substance that recognizes the target substance is preferred. When a substance that recognizes a target substance is a protein, a suitable material contains a macromolecular substance with high adsorptive properties, such as polystyrene or polymethyl methacrylate, as a main material. The shape of the support is not particularly limited. Preferably, the support is rod-shaped or plate-shaped, or shaped so as to have a depressed area(depressed areas) for confirmation of color development through visual inspection or the use of a color system measurement apparatus. In the case of a stick-rod-like shape or a plate-like shape, shapes having planar portions such as spatulate, stick-like, or plate-like shapes, or the like, are more suitable. In the case of such a shape having a depressed area(depressed areas), it should be able to avoid the spilling of liquids, so that incubation of a sample and an enzyme-labeled antibody can be performed. Furthermore, in terms of operation and procedures, a support preferably has a portion(portions) (to be measured) on which a target substance is immobilized and a portion(portions) (holding portion(s)) with which an operator (who performs measurement) holds the support. Moreover, materials for the holding portion(portions) are not particularly limited and may be any materials that are different from those of the measurement portion(portions). A support preferably has a space for recording the name of a sample.

An antibody against a target substance used in the present invention is not particularly limited, as long as it can specifically recognize the target substance, and as long as sites where the target substance is recognized by a substance immobilized on a support differ from sites where the target substance is recognized by an enzyme-labeled antibody. Any antibodies can be used, including polyclonal antibodies purified from antisera obtained by the immunization of mammals with target substances and monoclonal antibodies produced from hybridomas obtained by the fusion of myeloma cells with antibody-producing cells obtained from mammals immunized with target substances. A polyclonal antibody against a target substance can be obtained by purification with various means such as salting out, ion exchange chromatography, or affinity chromatography using antiserum obtained by the sensitization of mammals with the target substance as a material. A monoclonal antibody against a target substance can be purified by immunizing an appropriate animal such as a mouse with a target substance, fusing splenocytes to myeloma cells, selecting by techniques such as ELISA clones that produce an antibody that specifically binds to the target substance from the thus obtained hybridoma, and then purifying the monoclonal antibody using as a material the culture supernatant of the hybridoma or an ascite obtained by inoculating an appropriate animal with the hybridoma. The thus obtained antibody against a target substance can be used as a substance that recognizes the target substance and is immobilized on a support. Furthermore, an antibody may be labeled with an enzyme and can then also be used for recognizing a target substance bound to a support. As an enzyme-labeled antibody, a monoclonal antibody is preferably used for obtaining high selectivity for a target substance. When a substance that recognizes a target substance and is immobilized on a support is a monoclonal antibody, a polyclonal antibody can also be used as an enzyme-labeled antibody.

An enzyme-labeled antibody that can be used in the present invention is an antibody or an antibody fragment linked to an enzyme (an antibody labeled with an enzyme) and is used for the purpose of immunohistochemical staining, immunoassay, or the like. Enzymes used for labeling enzyme-labeled antibodies are not particularly limited. It is preferable to select horseradish peroxidase (hereinafter, abbreviated as HRP), alkaline phosphatase (hereinafter, abbreviated as ALP), or β-D-galactosidase (hereinafter abbreviated as β-Gal), because they are highly capable of converting substrates into products and are hardly deactivated.

There are two broad classifications of methods for labeling using enzymes: labeling methods using covalent bonds, and labeling methods using non-covalent bonds. Examples of a labeling method using covalent bonds include a method that involves linking the amino group of an antibody or an antibody fragment to the amino group of an enzyme, a method that involves previously binding an enzyme to an antibody at the gene level, so as to prepare an enzyme-labeled antibody, and a method that involves reducing an antibody or an antibody fragment so as to generate a thiol group of the hinge portion and then linking the resultant to an enzyme through the selective use of such thiol group. An example of a labeling method using non-covalent bonds involves previously causing an enzyme to react with an anti-enzyme antibody and then crosslinking the resultant with a target antibody using an antibody against such target antibody. Furthermore, an antibody may be previously labeled with biotin, following which the resultant is caused to react with enzyme-labeled avidin, so that an enzyme-labeled antibody can also be prepared. Such method for preparing an enzyme-labeled antibody of the present invention is not particularly limited. A preferable method involves performing reduction and then linking of the thiol group to an enzyme by a covalent bond, causing low levels of non-specific adsorption of a Fab' fragment of an enzyme-labeled antibody and preventing deterioration in the level of antibody activity.

In the present invention, a "substrate whose reaction product is water-insoluble after an enzyme-labeled antibody is caused to undergo color development" is a substance by which an enzyme-labeled antibody is caused to undergo color development, the reaction product produced after the color development of which is water-insoluble. A substrate whose reaction product is water-insoluble after an enzyme-labeled antibody is caused to undergo color development is not particularly limited. For example, when labeling with HRP is selected for an enzyme-labeled antibody, a solution preferable as a substrate contains 3-amino-9-ethylcarbazole, 5-aminosalicylic acid, 4-chloro-1-naphthol, o-phenylenediamine, 2,2'-azino-bis(3-ethylbenzthiazolin-6-sulfonic acid), 3,3-diaminobenzidine, 3,3', 5,5'-tetramethylbenzidine, o-dianisidine, or 3,3-dimethoxybenzidine. Furthermore, when labeling with ALP is selected for an enzyme-labeled antibody, a solution containing 5-bromo-4-chloro-3-indryl phosphate, nitroblue tetrazolium, or p-nitrophenyl phosphate can be used as a substrate. Furthermore, when labeling with β-Gal is selected for an enzyme-labeled antibody, a solution containing either o-nitrophenyl-β-D-galactoside or 5-bromo-4-chloro-3-indole-β-D-galactopyranoside can be used.

Color development can be detected visually or by measuring reflected light using a spectrophotometric colorimeter. In this case, the presence or the absence of a target substance can be determined or a target substance can be quantified by comparing color development between: a portion(portions) on a support, on which a substance that recognizes the target substance has been immobilized and a conjugated complex composed of a substance that recognizes a target substance, the target substance, and an enzyme labeled antibody has been formed as a result of a reaction with the target substance in a sample upon incubation; and a portion(portions) other than such portion(portions). Moreover, a standard curve is previously produced by plotting a concentration to reflected light using a sample containing a target substance with a known concentration. Following this, the concentration of the target substance in a sample may be found by measurement based on such curve.

Fig. 4 shows a step schematic diagram that is an embodiment of the measurement method of the present invention. The enzyme immunological kit of the present invention comprises at least a support upon the surface of which a substance that recognizes at least 1 type of target substance is immobilized, following which the surface is treated to inhibit non-specific adsorption of non-target substances, an enzyme-labeled antibody against the at least 1 type of target substance, a substrate whose reaction product is water-insoluble after the at least 1 type of enzyme-labeled antibody is caused to undergo color development, and at least 1 container for containing these substances. This means that an embodiment of the present invention is possible wherein: the 3 types of contents (a support coated with a substance that recognizes a target substance, the target substance, and an enzyme-labeled antibody against the target substance) are separately contained in different containers; the 3 types of such contents are contained in 1 container; or 2 types of contents (a support coated with a target substance and a substrate) or 2 types of contents (a support and an enzyme-labeled antibody) are contained in 1 container and the remaining 1 type of content is contained in another container. Subsequently, these containers are used for antigen-antibody reactions and color development reactions. The material of a container for containing 3 types of contents (a support coated with a substance that recognizes a target substance, the target substance, and an enzyme-labeled antibody against the target substance) and for causing reactions to take place among the same is not particularly limited. A container to which an enzyme-labeled antibody (that is, a protein) adheres with difficulty is preferable. The material of such container for containing 2 types of contents (a support coated with a target substance and a substrate) or 2 types of contents (a support and an enzyme-labeled antibody) and for causing reactions to take place among them is also not particularly limited. Such support and container preferably have spaces for recording the names of samples.

It is preferable for the enzyme immunological kit of the present invention to maintain its performance for 6 months or longer. To maintain performance for 6 months or longer, a conservation treatment method is preferably employed for a support on which a substance that recognizes a target substance has been immobilized, an enzyme-labeled antibody against the target substance, and a substrate that is used in the color development step. Decreased performance refers to as a decrease in detection sensitivity, mainly of a target substance. In the present invention, to avoid deterioration in a target-substance-recognizing substance immobilized on a support, a blocking agent, or an enzyme-labeled antibody, antiseptic finishing can be performed using a conservation solution such as a solution supplemented with thimerosal or sodium benzoate, potassium sorbate, sodium dehydroacetate, hinokitiol, butyl paraoxybenzoate, ε-polylysine, protamine, antibiotic, or the like, a high-concentration saccharose solution, or an NaCl solution. For antisepsis, sodium azide can also be used. A case where an HRP-labeled antibody is used is not preferable because such case causes a decrease in color development. An enzyme-labeled antibody against a target substance or a substrate that is used in the color development step can be sterilized by filtration when the molecular sizes thereof are smaller than the pore size of a filtration film. Furthermore, when there is no possibility of alteration in such antibody or substrate due to pressure or heat treatment, the solution can be sterilized by autoclave treatment. Moreover, deterioration in a kit's performance can be prevented by storing the kit within a rare gas or an inert gas environment, such as one comprised of incombustible nitrogen, or storing the same in a vacuum state. To avoid damage due to ultraviolet radiation, a light-resistant container is preferably used.

In the present invention, the concentration of a target substance with an unknown concentration in a sample can be measured by immobilizing a target-substance-recognizing substance on a support, causing a target substance with a known concentration to react with a portion(portions) of the support, incubating using the support a sample containing such target substance with an unknown concentration and an enzyme-labeled antibody against the target substance, performing a washing step, causing color development to take place using a substrate, and then comparing color development between the portion(portions) with which the target substance with the known concentration has been caused to react and a portion(portions) other than such portion(portions). With this method, the concentration of a target substance in a sample can be known more accurately. A portion(portions) with which a target substance with a known concentration is caused to react may be a portion(portions) coated with a substance that recognizes the target substance or a portion(portions) not coated with such substance that recognizes the target substance. Both types of portions may be used herein. In the subsequent incubation step, such portion(portions) must be caused to come into contact with an enzyme-labeled antibody, and then, in the color development step, it must be caused to come into contact with a substrate.

Through the use of the enzyme immunological kit and the measurement method according to the present invention, which comprises 2 reaction steps: an incubation step and a color development step wherein color development is caused to take place using a substrate whose reaction product is water-insoluble after an enzyme-labeled antibody is caused to undergo color development, disease can be rapidly diagnosed and a therapeutic method for patients can be determined within a short time. Such disease may be disease with an onset caused by an increase or decrease in a target substance, or a disease that causes an increase or decrease in a target substance, even if the target substance is not directly associated with the onset of such a disease.

Most cases involving such diseases that are problematic in terms of high levels of cytokines require treatment when the cytokine concentration is 500pg/ml or higher (Weekly Igaku No Ayumi (Journal of clinical and experimental medicine) 2001/1/6, Vol. 196, No. 1). Hence, it is important to know whether the cytokine concentration in a sample is 500pg/ml or higher. With the use of the measurement kit and the measurement method of the present invention, a cytokine with a concentration of 500pg/ml or higher in a sample can be detected. Therefore, hypercytokinemia can be diagnosed.

An example of a disease known to be characterized by high cytokine levels is a condition referred to as SIRS (Systemic Inflammatory Response Syndrome). The condition corresponds to a concept that has been proposed in regards to acute serious systemic inflammatory reactions. When 2 or more parameters, including body temperature, cardiac rate, respiration rate, and leukocyte count, are abnormal, a condition is determined to be a SIRS condition. It has been reported that organ failure is caused in the case of such condition by the action of inflammatory cytokines, and advancement of systemic inflammatory reactions results in death. As causative factors to induce SIRS, interleukins (hereinafter, abbreviated as IL), tumor necrosis factors (hereinafter, abbreviated as TNF), and interferons are known. In particular, the significant involvement of inflammatory cytokines such as IL-1, IL-2, IL-6, TNF, IL-8, and IFN-γ in SIRS has been suggested.

Cytokines are trace physiologically active substances significantly involved in immune reactions and inflammatory reactions. Cytokines are mainly produced from leukocytes. IL-1, IL-6, and TNF all induce acute phase reactions, so as to cause liver cells to synthesize and secrete a series of acute phase proteins, in addition to acting as endogenous pyrogens. IL-2 exerts various actions such as induction and activation of killer cells and B cell proliferation. IL-8 attracts neutrophils and T cells to inflammatory lesions. IFN-γ activates macrophages and promotes active oxygen production.

It has also been reported that severity correlates with the blood cytokine concentrations of patients with pancreatitis. An example of the cause of such condition is a vicious cycle whereby neutrophils activated by cytokines destroy organs, following which cytokines are further induced by such stimulation (Ishiyaku Publishers, Inc., separate volume, *Igaku No Ayumi* (Journal of clinical and experimental medicine), Cytokine-from the basics to clinical applications). Furthermore, it has been reported that when the blood IL-6 concentration is high in a severe acute pancreatitis case, there is a high risk of increasing severity (e.g., development of complications such as organ failure) ("The Journal of the Japanese Association for Acute Medicine," Volume 11, Number 10, October 2000).

In the case of hemophagocytic syndrome, proliferation of lymphoid cells results in a condition of hypercytokinemia. Cytokines enhance the phagocytic activity of lymphocytes, monocytes, and the like, thereby destroying hemocytes. When the severity is increased, multiple organ failure and the like may also be induced. Diagnosis of such disease is difficult, and the causes thereof or therapeutic methods therefor are under research.

When IL-8 is injected intraperitoneally or intravenously, neutrophils in peripheral blood increase within 1 hour. When IL-8 is administered subcutaneously or into an articular cavity, recruitment of large numbers of neutrophils and T lymphocytes is observed at administered sites. In particular, sustained intravenous administration of IL-8 induces destruction of pulmonary alveoli, resulting in a condition that resembles the acute respiratory distress syndrome (hereinafter, abbreviated as ARDS). Administration of IL-8 into the articular cavity may also exert strong action to cause organ dysfunction, such that destruction of synovial membranes is observed. Also, IL-8 is detected *in vivo* in connection with diseases such as gout, chronic rheumatism, pyelonephritis, asthma, sepsis, ARDS, Crohn's disease, and ulcerative colitis.

In addition to such diseases, it is thought that hypercytokinemia is also associated with Kawasaki's disease accompanied by angitis, and immune system diseases such as collagen disease and Still's disease. Furthermore, it is thought that cytokines in amniotic fluids in cases of threatened premature delivery cases are useful markers for indicating premature labor (Ginekol Pol. 2003, Oct; 74(10): 1343-7).

Furthermore, it has also been demonstrated that after invasion associated with injuries, cytokine production due to overactivity of immunocytes induces SIRS and causes organ dysfunction. It has been reported that when a state of shock is reproduced by *in vivo* administration of inflammatory cytokines and hypercytokinemia is continued after injuries have been received, such condition correlates significantly with the coincidence of infectious disease and mortality based on such coincidence. It is thought that 50% or more of injury deaths are not caused by irreversible nerve damage or massive hemorrhaging immediately after accidents, but rather by sepsis or multiple organ failure (MOF) following such damage or hemorrhaging. Regarding burn models, it has been reported that exotoxins enhance early T cell reactions, thereby inducing lethal shock-like pathological conditions. Such result demonstrates the fact that immune responses are elevated in SIRS pathological conditions. This is a report that such elevation is involved in the increased severity of pathological conditions. It is thought that such conditions clinically correspond to ARDS or TSS, whereby basically no infection is observed in defective organs and important organs are damaged by immunocytes activated in manners similar to those in cases of autoimmune diseases.

Furthermore, it is known that disease caused by infection may also lead to hypercytokinemia. When no clear infection is observed, the immune system can be over-activated by superantigens produced by *Staphylococcus aureus,* group A-β hemolytic streptococcus, or the like. Since such cases may lead to hypercytokinemia, early diagnosis is important. Examples of diseases in which superantigens are involved include TSS, NTED, systemic *Yersinia enterocolitica* infection, scarlatina, and STSS. The involvement of a superantigen in Kawasaki's disease, which is acute systemic angiitis, has also been suggested. For these diseases, it is important to avoid increases in severity by early and rapid diagnosis and the initiation of treatment based on the thus made diagnosis. In particular, STSS begins with pharyngeal pain, fervescence, digestive system symptoms (e.g., anorexia, nausea, vomiting, and diarrhea), septic symptoms such as low blood pressure, or symptoms of myalgia. Such symptoms proceed acutely, resulting in soft tissue lesion, circulatory and/or respiratory failure, abnormal blood coagulation, or multiple organ failure such as in the case of renal and/or liver symptoms within 24 hours. Unless appropriate treatment is performed early, the outcomes can include multiple organ failure, necrosis of extremities, and death, for example. The presence of the causative bacteria of these infectious diseases can also be proved by culturing such bacteria. However, culture of bacteria often requires 1 or more weeks. Furthermore, regarding rapid detection of infection with group A-β hemolytic streptococcus, JP Patent Publication (Kokai) No. 6-50973 A (1994) discloses a measurement method for a Strep A antigen. However, the detection subject in this method is limited to group A β-hemolytic streptococcus, and the concentration of a superantigen in a specimen cannot be measured with this method. Kits for rapidly detecting infection with *Staphylococcus aureus* have also been marketed. However, the subjects of these kits are bacteria after culture.

When blood cytokine and superantigen concentrations are at high levels, patient prognosis depends a great deal upon the rapid handling of such conditions and the realization of rapid decreases in such concentrations of problematic substances. Furthermore, the measurement of cytokines or superantigens currently requires complicated techniques (e.g., culture of bacteria and ELISA) and much time. Hence, a measurement method capable of rapidly and conveniently measuring such concentrations within an extremely short period of time at bedside has been desired. Even when it is difficult to make a diagnosis, easy examination of the concentrations of substances that are associated with these diseases enables early and appropriate treatment of patients without overlooking such diseases.

### Examples

The present invention will be described in detail as follows using examples, but the content of the present invention is not limited to such examples.

### Example 1: Immobilization of an anti-IL-8 antibody on a support, followed by blocking and conservation treatment

An anti-human IL-8 polyclonal antibody was prepared at 2 µg/ml using phosphate buffered saline. 1 ml of the solution was pipetted to a polypropylene container. A support (produced by Nunc) made of polystyrene was immersed in the solution and then allowed to stand at 4°C for 18 hours, so that the anti-IL-8 antibody was immobilized on a stick.

Subsequently, the solution that had adhered to the container and to the stick was removed. The support was immersed in 1 ml of phosphate buffered saline supplemented with 0.5% bovine serum albumin (produced by Serological) and then allowed to stand at room temperature (22°C) for 2 hours, thereby resulting in blocking treatment. Subsequently, the solution that had adhered to the container and the stick was removed. The resultant was immersed in 1 ml of a phosphate buffer solution containing 30% saccharose and then subjected to conservation treatment. Subsequently, the solution that had adhered to the container and the support was removed. An anti-human IL-8 polyclonal antibody used herein was prepared by obtaining antiserum through subcutaneous administration of natural IL-8 derived from human fibroblasts to a rabbit and then subjecting the antiserum to affinity purification using a column in which IL-8 had been immobilized.

### Example 2: Incubation step, water washing step, and color development step

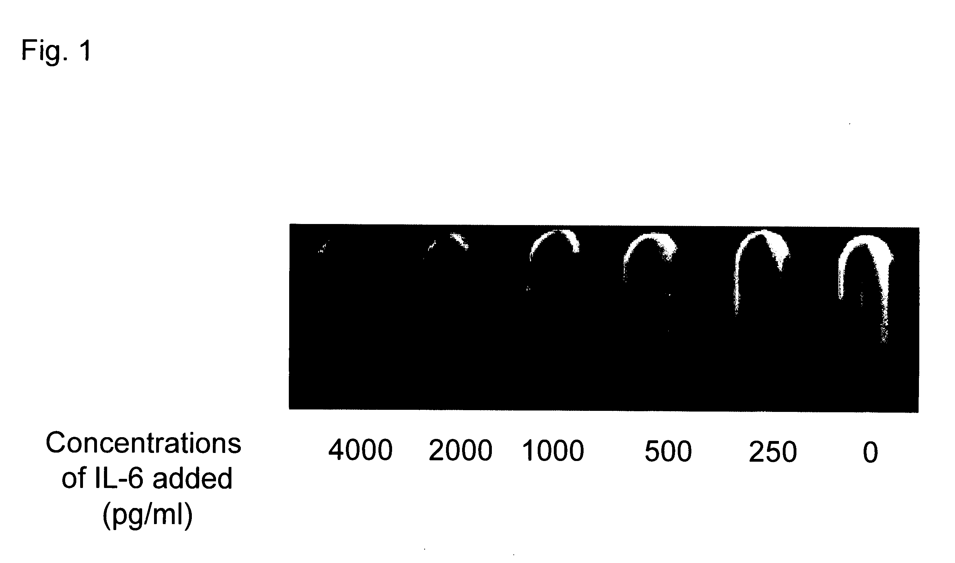

An HRP-labeled anti-IL-8 monoclonal antibody was prepared at 0.5 µg/ml using phosphate buffered saline supplemented with 0.25% bovine serum albumin (produced by Serological) and 0.05% Tween 20 (produced by Tokyo Chemical Industry). The solution was pipetted in amounts of 250µl each to containers made of polypropylene. The HRP-labeled monoclonal antibody had been obtained by labeling with HRP the monoclonal antibody obtained by culturing EL139 (deposited under accession No. FERM P-12710 with the International Patent Organism Depositary (IPOD), the National Institute of Advanced Industrial Science and Technology (AIST) (Chuo 6, Higashi 1-1-1, Tsukuba-shi, Ibaraki, Japan)). Human plasma samples (IL-8 concentrations had already been confirmed by ELISA (produced by Biosource) to be at or lower than the level of detection sensitivity) prepared to contain 4ng/ml, 2 ng/ml, 1ng/ml, 0.5ng/ml, 0.25ng/ml, and 0ng/ml human IL-8 (produced by Biosource) were each separately pipetted in amounts of 250µl each to different containers. The supports of the type prepared in Example 1 were immersed separately in each solution and then allowed to stand for 30 minutes at room temperature (2°C). The supports were used 41 days after the procedures of Example 1. At this time, the area of the portion(portions) of the support surface on which the anti-human IL-8 polyclonal antibody had been immobilized per ml of each human plasma sample was 6.7cm²/ml.. Subsequently, the solutions adhered in the containers and to the supports were removed, followed by washing with distilled water. 1 ml of a substrate (produced by Promega) containing 3,3',5,5'-tetramethylbenzidine was pipetted into each container. The solutions were allowed to stand for 15 minutes at room temperature. Subsequently, the solutions adhered in the container and to the supports were removed, followed by washing with distilled water. The degree of color development on each support was confirmed by visual inspection and the L*a*b* color system. The L* value, a* value, and b* value at each IL-8 concentration were measured using a spectrophotometric colorimeter (CM-2600d produced by KONICA MINOLTA) and then compared with the L* value, a* value, and b* value at 0 ng/ml. Fig. 1 is a photograph showing the degrees of color development on the supports resulting from the human plasma samples to which IL-8 had been added. Table 1 shows ΔL* values, Δa* values, and Δb* values.

**Table 1**

| Color development on the supports resulting from the human plasma samples to which IL-8 was added (Quantification based on the L*a*b* color system) | | | | | |
|---|---|---|---|---|---|
| Concentrations of IL-8 added (pg/ml) | 4000 | 2000 | 1000 | 500 | 250 |
| ΔL* | -13.6 | -4.63 | -4.29 | -1.64 | 0.04 |
| Δa* | -10.9 | -10.3 | -5.77 | -3.06 | -0.51 |
| Δb* | -4.24 | -1.82 | 0.99 | 0.68 | 0.20 |

### Example 3: Immobilization of an anti-IL-6 antibody onto a support, followed by blocking and conservation treatment

An anti-human IL-6 polyclonal antibody was prepared at 2µg/ml using phosphate buffered saline. 1 ml of the solution was pipetted to a polypropylene container. A support (produced by Nunc) made of polystyrene was immersed in the solution and then allowed to stand at 4°C for 18 hours, so that the anti-IL-6 antibody was immobilized on a stick. Subsequently, the solution that had adhered to the container and the stick was removed. The support was immersed in 1 ml of phosphate buffered saline supplemented with 0.5% bovine serum albumin (produced by Serological) and then allowed to stand at room temperature (22°C) for 2 hours, thereby performing blocking treatment. Subsequently, the solution that had adhered to the container and the stick was removed. The resultant was immersed in 1 ml of a phosphate buffer solution containing 30% saccharose and then subjected to conservation treatment. Subsequently, the solution that had adhered to the container and the support was removed. An anti-human IL-6 polyclonal antibody used herein was prepared by obtaining antiserum through subcutaneous administration of natural IL-6 derived from human fibroblasts to a goat and then subjecting the antiserum to affinity purification using a column in which IL-6 had been immobilized.

### Example 4: Incubation step, water washing step, and color development step

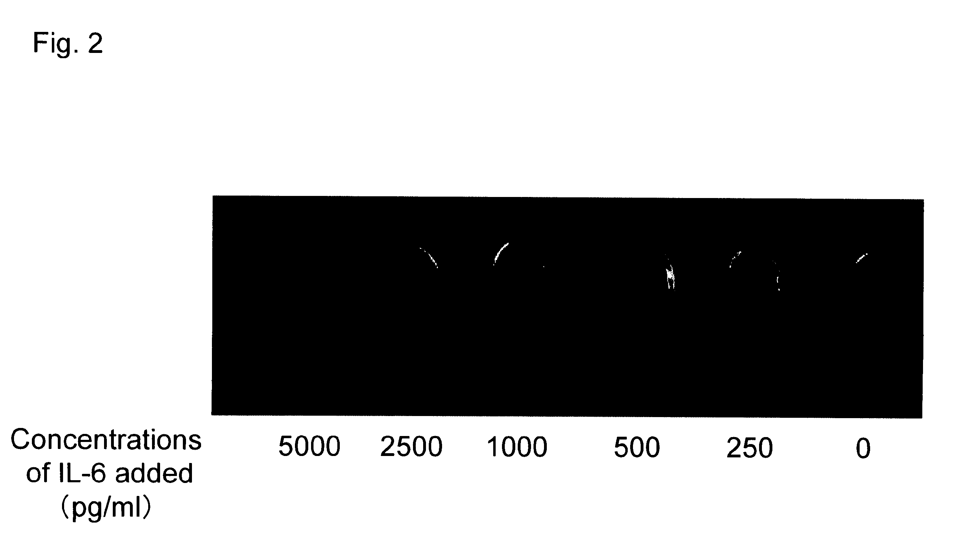

An HRP-labeled anti-IL-6 monoclonal antibody was prepared at 0.5µg/ml using phosphate buffered saline supplemented with 0.25% bovine serum albumin (produced by Serological) and 0.05% Tween 20 (produced by Tokyo Chemical Industry). The solution was pipetted in amounts of 250µl each to containers made of polypropylene. The HRP-labeled monoclonal antibody had been obtained by labeling with HRP the monoclonal antibody obtained by culturing IG61 (deposited under accession No. FERM P-10713 (International Deposition No. 2878) with the International Patent Organism Depositary (IPOD), the National Institute of Advanced Industrial Science and Technology (AIST)). Human plasma samples (IL-6 concentrations had already been confirmed by ELISA (produced by Biosource) to be at or lower than the level of detection sensitivity) prepared to contain 5ng/ml, 2.5ng/ml, 1 ng/ml, 0.5ng/ml, 0.2 ng/ml, and 0ng/ml human IL-6 (produced by Biosource) were each separately pipetted in amounts of 250µl each to different containers. The support prepared in Example 1 was immersed in each solution and then allowed to stand for 30 minutes at room temperature (22°C). The support was used 41 days after the procedures of Example 1. At this time, the area of the portion(portions) of the support surface on which the anti-human IL-6 polyclonal antibody had been immobilized per ml of each human plasma sample was 6.7cm²/ml. Subsequently, the solutions adhered in the containers and to the supports were removed, followed by washing with distilled water. 1 ml of a substrate (produced by Promega) containing 3,3',5,5'-tetramethylbenzidine was pipetted into each container. The solutions were allowed to stand for 15 minutes at room temperature. Subsequently, the solutions that had adhered in the container and to the supports were removed, followed by washing with distilled water. The degree of color development on each support was confirmed by visual inspection and the L*a*b* color system. The L* value, a* value, and b* value at each IL-6 concentration were measured using a spectrophotometric colorimeter (CM-2600d produced by KONICA MINOLTA) and then compared with the L* value, a* value, and b* value at 0 ng/ml. Fig. 2 is a photograph showing the degrees of color development on the supports resulting from the human plasma samples to which IL-6 had been added. Table 2 shows ΔL* values, Δa* values, and Δb* values.

**Table 2**

| Color development on the supports resulting from the human plasma samples to which IL-6 was added (Quantification based on the L*a*b* color system) | | | | | |
|---|---|---|---|---|---|
| Concentrations of IL-6 added (µg/ml) | 5000 | 2500 | 1000 | 500 | 250 |
| ΔL* | -15.0 | -7.62 | -3.55 | -1.78 | 0.10 |
| Δa* | -11.2 | -9.83 | -5.01 | -2.04 | -0.32 |
| Δb* | -4.74 | -2.09 | 1.03 | 0.22 | 0.21 |

It was thus demonstrated that the degrees of color development on the supports differ depending on the concentrations of cytokines, that human cytokine concentrations correlate with L* values, a* values, and b* values, and that only when a cytokine concentration in a sample is 500pg/ml or more color development can be confirmed.

### Comparative Example 1: The degree of color development differing depending on the amounts of antibodies against a target substance immobilized on supports

The anti-human IL-8 polyclonal antibody in Example 1 and the anti-human IL-6 polyclonal antibody in Example 3 were prepared at concentrations of 0.05pg/ml and 25µg/ml, respectively. Procedures following the preparation were performed according to Examples 1 to 4, except for using as samples human plasma samples (IL-6 and IL-8 concentrations had already been confirmed by ELISA (produced by Biosource) to be at or lower than the level of detection sensitivity) prepared to contain IL-6 at 0.5ng/ml and IL-8 at 0.5ng/mi.

As described above, when the immobilization amounts of the antibodies against the target substance onto the supports were too low or too high, the degrees of color development were lowered. Hence, color development characterized by the cytokine concentration of 500µg/ml in a sample could not be confirmed.

### Comparative Example 2: The degree of color development differing depending on the concentrations of enzyme-labeled antibodies against a target substance

The HRP-labeled anti-IL-8 monoclonal antibody in Example 2 and the HRP-labeled anti-IL-6 monoclonal antibody in Example 4 were prepared at 0.05µg/ml and 25µg/ml, respectively. Procedures following the preparation were performed according to Examples 1 to 4, except for using as samples human plasma samples (IL-6 and IL-8 concentrations had already been confirmed by ELISA (produced by Biosource) to be at or lower than the level of detection sensitivity) prepared to contain IL-6 at 0.5ng/ml and IL-8 at 0.5ng/ml.

As described above, when the concentrations of the enzyme-labeled antibodies against the target substance were too low, color development characterized by a cytokine concentration of 500pg/ml in a sample could not be confirmed. When the concentrations of the same antibodies were too high, it was confirmed that non-specific color development took place, even in a sample containing no cytokines.

### Example 5: Cytokine measurement in hypercytokinemia patient's blood

The degrees of color development on supports were confirmed using the serum (with a known cytokine concentration) of a hypercytokinemia patient instead of using the human plasma samples supplemented with human IL-8 of Example 2.

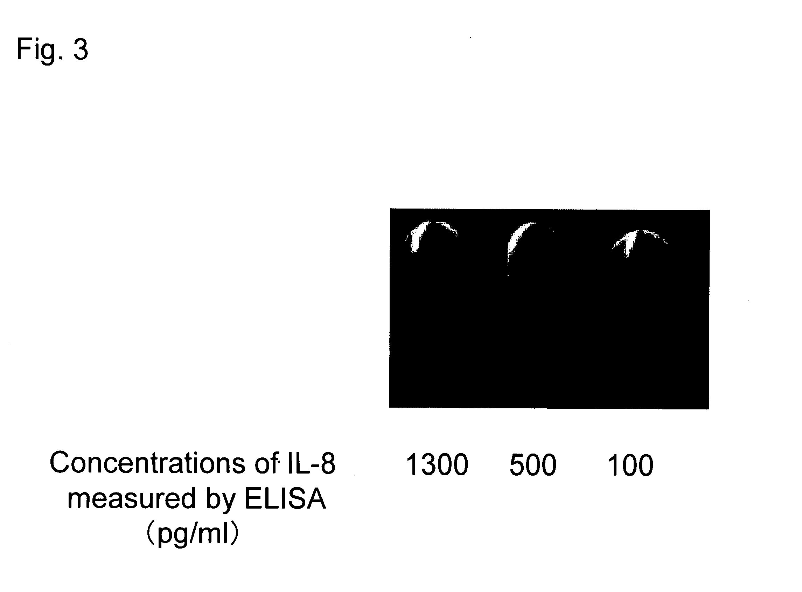

The cytokine concentration in the patient's blood was confirmed by ELISA (produced by Biosource). Fig. 3 is a photograph showing color development (quantification based on the L*a*b* color system) on the supports resulting from IL-8 in the patient's serum. Table 3 shows ΔL* values, Δa* values, and Δb* values.

**Table 3**

| Color development on the supports resulting from the cytokine in the patient's serum (Quantification based on the L*a*b* color system) | | | |
|---|---|---|---|
| Concentrations of IL-8 added (µg/ml) | 1300 | 500 | 100 |
| ΔL* | -4.33 | -2.20 | 0.06 |
| Δa* | -6.09 | -4.45 | -0.11 |
| Δ b* | -1.66 | 0.44 | 0.12 |

As described above, blood cytokine concentrations could be measured through comparison with color development on the supports of Example 2. Thus, hypercytokinemia could be diagnosed.

### Industrial Applicability

The enzyme immunological kit of the present invention is capable of rapidly and easily measuring a target substance with 2 procedures based on the color development on a support. Furthermore, a substance that recognizes a target substance is immobilized on the above support surface at a concentration of 2cm² or more per ml of a sample. Since the concentration of the target substance in the sample has a linear correlation with the strength of color development, the target substance can be accurately quantified.

Therefore, the presence or the absence of a target substance can also be determined by determination of the presence or the absence of color development. Moreover, the precise concentration of a target substance can also be measured.

Furthermore, with the enzyme immunological kit of the present invention, cytokines, *Staphylococcus aureus* exotoxin and Streptococcus group A exotoxin can be detected. Furthermore, hypercytokinemia, disease caused by *Staphylococcus aureus,* and disease caused by group A β-hemolytic streptococcus can be rapidly, easily, and accurately diagnosed.

All publications cited herein are incorporated herein in their entirety. A person skilled in the art would easily understand that various modifications and changes of the present invention are feasible within the technical idea and the scope of the invention as disclosed in the attached claims. The present invention is intended to include such modifications and changes.

## Claims

1. An enzyme immunological kit, which comprises: a support upon the surface of which a substance that recognizes at least 1 type of target substance is immobilized, following which the surface is treated to inhibit non-specific adsorption of non-target substances; an enzyme-labeled antibody against the at least 1 type of target substance; a substrate whose reaction product is water-insoluble after the at least 1 type of enzyme-labeled antibody is caused to undergo color development; and at least 1 container for receiving the aforementioned items; and an antibody-antigen reaction and a color development reaction are caused to take place in the container and the target substance in a sample is detected based on color development on the support surface on which the substance that recognizes the target substance is immobilized at a concentration of 2cm² or more per ml of the sample.

2. An enzyme immunological kit, which comprises: a support upon the surface of which a substance that recognizes at least 1 type of target substance is immobilized, following which such surface is treated to inhibit non-specific adsorption of non-target substances, and then undergoes a reaction of the at least 1 type of target substance with a known concentration on such surface; an enzyme-labeled antibody against the at least 1 type of target substance; a substrate whose reaction product is water-insoluble after the at least 1 type of enzyme-labeled antibody is caused to undergo color development; and at least 1 container for receiving the aforementioned items; and with which antibody-antigen and color development reactions are caused to take place in the container and the concentration of the target substance with an unknown concentration in a sample is measured through comparison of color development between a portion(portions) where the target substance with the known concentration reacts and portions other than such portion(portions) on the support surface on which the substance that recognizes the target substance is immobilized at a concentration of 2cm² or more per ml of the sample.

3. The enzyme immunological kit according to claim 2, by which, simultaneously with detection of the target substance in the sample based on color development on the support surface, the concentration of the target substance with the unknown concentration in the sample is measured through comparison of color development between the portion(portions) where the target substance with the known concentration reacts and the portions other than such portion(portions) on the support surface.

4. The enzyme immunological kit according to any one of claims 1 to 3, wherein the substance that recognizes the target substance is immobilized on the surface of the support through contact of a solution containing the substance that recognizes the target substance with the support, and wherein the concentration of the substance that recognizes the target substance in the solution ranges from 0.1 to 20µg/ml when immobilized.

5. The enzyme immunological kit according to claim 4, wherein pH ranges from 6.5 to 8.0 when immobilized.

6. The enzyme immunological kit according to claim 4 or 5, wherein the concentration of the enzyme-labeled antibody against the target substance ranges from 0.1 to 20µg/ml.

7. The enzyme immunological kit according to any one of claims 1 to 6, wherein the target-substance-recognizing substance immobilized on the support is immobilized in an amount that causes the establishment of a linear correlation between the concentration of the target substance in the sample and the strength of color development.

8. The enzyme immunological kit according to any one of claims 1 to 7, wherein the substance that recognizes the target substance is an antibody against the target substance and the antibody is a polyclonal antibody.

9. The enzyme immunological kit according to any one of claims 1 to 8, wherein the activity of the substance that recognizes the target substance is not lowered until 6 months after treatment with an antisepsis agent, pressure or heat treatment, or storage with an inert gas or in a vacuum state.

10. The enzyme immunological kit according to any one of claims 1 to 9, wherein the support is rod-shaped, plate-shaped, or shaped so as to have a depressed area(depressed areas).

11. The enzyme immunological kit according to any one of claims 1 to 10, wherein the enzyme-labeled antibody is a monoclonal antibody.

12. The enzyme immunological kit according to claim 11, wherein the monoclonal antibody is an antibody against a human interleukin.

13. The enzyme immunological kit according to claim 11 or 12, wherein the monoclonal antibody is IgG.

14. The enzyme immunological kit according to claim 13, wherein the monoclonal antibody is IgG

15. The enzyme immunological kit according to any one of claims 12 to 14, wherein the human interleukin is IL-6 or IL-8.

16. The enzyme immunological kit according to claim 15, wherein the monoclonal antibody recognizes a fragment comprising the 149^{th} to the 173 rd amino acids of the amino acid sequence of human IL-6.

17. The enzyme immunological kit according to claim 15, wherein the monoclonal antibody recognizes a fragment comprising the 153^{rd} to the 162^{nd} amino acids of the amino acid sequence of human IL-6.

18. The enzyme immunological kit according to claim 16, wherein the antibody against human IL-6 is a clone IG61 produced by the hybridoma deposited under accession No. FERM P-10713 with the International Patent Organism Depositary (IPOD), the National Institute of Advanced Industrial Science and Technology (AIST).

19. The enzyme immunological kit according to claim 17, wherein the antibody against human IL-8 is a clone EL139 produced by the hybridoma deposited under accession No. FERM P-12710 with the International Patent Organism Depositary (IPOD), the National Institute of Advanced Industrial Science and Technology (AIST).

20. The enzyme immunological kit according to any one of claims 1 to 19, wherein the enzyme for the enzyme-labeled antibody is horseradish peroxidase, alkaline phosphatase, or β-D-galactosidase.

21. The enzyme immunological kit according to any one of claims 1 to 20, wherein the enzyme for the enzyme-labeled antibody is horseradish peroxidase and the substrate is a solution containing 3-amino-9-ethylcarbazole, 5-aminosalicylic acid, 4-chloro-1-naphthol, o-phenylenediamine, 2,2'-azino-bis(3-ethylbenzothiazolin-6-sulfonic acid), 3,3-diaminobenzidine, 3,3', 5,5'-tetramethylbenzidine, o-dianisidine, or 3,3-dimethoxybenzidine.

22. The enzyme immunological kit according to any one of claims 1 to 21, wherein the enzyme for the enzyme-labeled antibody is alkaline phosphatase and the substrate is a solution containing 5-bromo-4-chloro-3-indryl phosphate, nitroblue tetrazolium, or p-nitrophenyl phosphate.

23. The enzyme immunological kit according to any one of claims 1 to 20, wherein the enzyme for the enzyme-labeled antibody is β-D-galactosidase and the substrate is a solution containing either o-nitrophenyl-β-D-galactoside or 5-bromo-4-chloro-3-indole-β-D-galactopyranoside.

24. The enzyme immunological kit according to any one of claims 1 to 23, wherein the target substance is a cytokine, *Staphylococcus aureus* exotoxin, or Streptococcus group A exotoxin.

25. The enzyme immunological kit according to claim 24, wherein the cytokine is an inflammatory cytokine.

26. The enzyme immunological kit according to claim 25, wherein the inflammatory cytokine is IL-1, IL-2, IL-4, IL-6, IL-8, TNF-α, or IFN-γ.

27. The enzyme immunological kit according to any one of claims 24 to 26, wherein the detection limit concentration of the cytokine is 500pg/ml.

28. The enzyme immunological kit according to claim 24, wherein the *Staphylococcus aureus* exotoxin is a superantigen.

29. The enzyme immunological kit according to claim 28, wherein the superantigen is SEA, SEB, SEC, SED, SEE, SEH, SEI, SEJ, or TSST-1.

30. The enzyme immunological kit according to claim 24, wherein the Streptococcus group A exotoxin is a superantigen.

31. The enzyme immunological kit according to claim 30, wherein the superantigen is SPEA, SPEC, SPEF, SPEG, or SPEH.

32. The enzyme immunological kit according to any one of claims 1 to 27, which is used for rapidly determining whether a disease is caused by hypercytokinemia.

33. The enzyme immunological kit according to any one of claims 1 to 24, 28, and 29, which is used for rapidly determining whether a disease is caused by *Staphylococcus aureus.*

34. The enzyme immunological kit according to any one of claims 1 to 24, 30, and 31, which is used for rapidly determining whether a disease is caused by group A β-hemolytic streptococcus.

35. The enzyme immunological kit according to any one of claims 1 to 35, which comprises converting color development on the support surface into numerical values using a measurement apparatus and then quantifying the color development using the numerical values, wherein light reflecting from the support surface is dispersed within a range between 400nm and 700nm, the reflectance at every each wavelength is measured, and parameters based on the XYZ or L*a*b*color system are thus measured.

36. A method for detecting a target substance in a sample or measuring the concentration of a target substance in a sample, wherein the enzyme immunological kit according to any one of claims 1 to 35 is used.

37. A method for detecting a target substance in a sample, which comprises 2 reaction steps: the incubation step for simultaneously incubating a support having a portion(portions) coated with a substance that recognizes a target substance, a sample, and an enzyme-labeled antibody against the target substance; and the color development step for causing color development to take place using a substrate whose reaction product is water-insoluble, wherein in the incubation step, the substance that recognizes the target substance is added to the support surface at a concentration of 2 cm² or more per ml of the sample, and the color of the support surface is visually determined after the color development reaction.

38. The method for detecting a target substance in a sample according to claim 37, wherein the concentration of the substance that recognizes the target substance in the solution ranges from 0.1 to 20µg/ml and the pH ranges from 6.5 to 8.0 when the substance is immobilized on the support having a portion(portions) coated with the substance that recognizes the target substance,.

39. The method for detecting a target substance in a sample according to claim 37 or 38, wherein the concentration of the enzyme-labeled antibody against the target substance, which is used in the color development step, ranges from 0.1 to 20µg/ml.

40. The method according to any one of claims 37 to 39, which is a method for measuring the concentration of a target substance in a sample, wherein the color of the support surface is visually determined after the color development reaction.

41. The method according to any one of claims 37 to 39, which is a method for detecting a target substance in a sample, wherein the color of the support surface is converted into numerical values using an apparatus after the color development reaction, and then the color development is quantified using the numerical values.

42. The method according to any one of claims 36 to 41, which is a method for detecting a target substance in a sample, wherein light reflecting from a sample is dispersed within a range between 400nm and 700nm, the reflectance is measured at every each wavelength, and then parameters based on a color system are measured, and the color system is the XYZ or L*a*b* system.

43. The method for detecting a target substance in a sample according to any one of claims 37 to 42, which comprises a washing step between the incubation step and the color development step.

44. The method for detecting a target substance in a sample according to any one of claims 37 to 43, wherein the reaction temperature for the incubation, washing, or color development step is within the range between 4°C and 40°C.

45. The method for detecting a target substance in a sample according to claim 44, wherein the incubation step, the color development step, and the washing step are performed at room temperature.

46. The method for detecting a target substance in a sample according to any one of claims 37 to 45, wherein the amount of a sample is 200µl or less.

47. The method for detecting a target substance in a sample according to any one of claims 37 to 46, wherein the time spent for the incubation step is within the range between 5 and 60 minutes and the time spent for the color development step is within the range between 5 and 30 minutes.

48. The method for detecting a target substance in a sample according to any one of claims 37 to 47, which comprises causing at least 1 type of target substance with a known concentration to react with at least either a part of a portion(portions) coated with or a part of a portion(portions) not coated with the substance that recognizes the target substance on the support having such portion(portions) coated with the substance that recognizes the target substance, and then comparing color development between a portion(portions) where the target substance with the known concentration reacts and portions other than such portion(portions), thereby measuring the concentration of the target substance with an unknown concentration in a sample.

49. The method according to any one of claims 37 to 48, wherein the support having portions coated with the substances that recognizes target-substances is coated with the target-substance-recognizing substance at at least two different concentrations and the concentration of a target substance in a sample is measured by comparing the degree of color development between at least 2 portions.

50. The method according to any one of claims 37 to 49, wherein the target substance is a cytokine and the detection limit concentration thereof is 500 pg/ml.

51. A method for diagnosing hypercytokinemia through detection of a cytokine using the enzyme immunological kit according to any one of claims 24 to 27 and 32.

52. A method for diagnosing a disease caused by *Staphylococcus aureus* through detection of *Staphylococcus aureus* exotoxin using the enzyme immunological kit according to any one of claims 28, 29, and 33.

53. A method for diagnosing a disease caused by group A β-hemolytic streptococcus through detection of group A β-hemolytic streptococcus exotoxin using the enzyme immunological kit according to any one of claims 30, 31, and 34.
